**Europäisches Patentamt**

**European Patent Office**

**Office européen des brevets**

(11) Veröffentlichungsnummer: **0 039 443**
A1

# EUROPÄISCHE PATENTANMELDUNG

(21) Anmeldenummer: **81103029.5**

(22) Anmeldetag: **22.04.81**

(51) Int. Cl.³: **A 61 F 7/00,** A 61 B 5/00

(30) Priorität: **24.04.80 DE 3015787**

(43) Veröffentlichungstag der Anmeldung: **11.11.81**
**Patentblatt 81/45**

(84) Benannte Vertragsstaaten: **AT BE CH FR GB IT LI LU NL SE**

(71) Anmelder: **Steingraf, Peter, Schleussnerstrasse 26, D-6380 Bad Homburg v. d. H. (DE)**

(72) Erfinder: **Steingraf, Peter, Schleussnerstrasse 26, D-6380 Bad Homburg v.d.H. (DE)**
Erfinder: **Sdunzig, Horst, Neugasse 10, D-6203 Hochheim-Massenheim (DE)**
Erfinder: **Hartmann, Rolf, Am Hohenstein 2, D-6233 Kelkheim (DE)**

(74) Vertreter: **Blum, Klaus-Dieter, Dipl.-Ing., c/o Battelle-Institut e.V. Am Römerhof 35, D-6000 Frankfurt/Main (DE)**

(54) **Vorrichtung zur Kühlung von Körperstellen oder Körperteilen im Rahmen therapeutischer oder diagnostischer Massnahmen.**

(57) In einer Vorrichtung zur Kühlung von Körperstellen oder Körperteilen bestehend im wesentlichen aus einer von einem Wärmetransportmedium durchflossenen Auflage oder Bandage, einem Kühlaggregat und aus Meß- und Regelungseinrichtungen zur Steuerung des Wärmetransportes werden Einrichtungen (7, 8, 20) zur Messung der Temperatur und/oder der Wärmeableitung von der behandelnden Körperstelle vorgesehen. Die Wärmeableitung ist in Abhängigkeit von einer Vergleichstemperatur derart steuerbar, daß gerade nur die überschüssige Wärmemenge abgeführt wird. Die Kühlung ist selbsttätig ausschaltbar, sobald die Wärmeerzeugung an der behandelten Körperstelle bzw. die Wärmeableitung auf einem Vergleichswert oder Sollwert zurückgegangen ist. Der Vergleichswert oder Sollwert kann mit Hilfe einer zusätzlichen, an einem äquivalenten, jedoch gesunden Körperteil anlegbaren Temperaturmeßelektrode (22) ermittelt werden.

V-64.053-01/57/79

CASCH/KRI                                    18. April 1980

PETER STEINGRAF, BAD HOMBURG

=================================================================
Vorrichtung zur Kühlung von Körperstellen oder Körperteilen
   im Rahmen therapeutischer oder diagnostischer Maßnahmen
=================================================================

Die Erfindung bezieht sich auf eine zur Kühlung von Körperstellen oder Körperteilen im Rahmen therapeutischer oder diagnostischer Maßnahmen vorgesehene Vorrichtung, die im wesentlichen aus einer von einem Wärmetransportmedium durchflossenen Auflage oder Bandage, einem Kühlaggregat und aus Meß- und Regelungseinrichtungen zur Steuerung des Wärmetransportes besteht.

Es ist bereits seit langem bekannt, daß durch Kühlen von Körperstellen bei bestimmten Erkrankungen, z.B. Entzündungen, Heilerfolge erzielt oder Schmerzen gelindert werden können. Im einfachsten Fall werden zum Kühlen kalte Wickel verwendet, die

-2-

jedoch häufig erneuert werden müssen, wenn eine länger anhaltende Kühlung erreicht werden soll. Eisbeutel oder medizinische Bäder sind ebenfalls nur für relativ kurzzeitige Anwendung geeignet, wenn man von ständiger Erneuerung absieht. Die genaue Einstellung und Einhaltung von aus therapeutischer Sicht optimalen Kühltemperaturen bereitet gleichfalls große Schwierigkeiten oder ist mit den bekannten und üblichen Hilfsmitteln kaum möglich.

Die gezielte Absenkung der Temperatur über längere Zeitspannen hinweg und die Einhaltung bestimmter, aus medizinischer Sicht optimaler oder zumindest zweckmäßiger Temperaturen bereitet dagegen große Schwierigkeiten. Bei Verwendung von Eis, z.B. in Eisbeuteln, liegt die Anfangstemperatur zwangsläufig unter $0^{o}C$ und steigt dann unter dem Einfluß der Körperwärme kontinuierlich an. Außerdem stört in der Praxis die schwierige Handhabung des starren Eises, das ohne Zerkleinerung nicht in eine anschmiegsame Bandagenform gebracht werden kann. Man nimmt daher häufig anstelle von Wasser Gemische mit tieferem Erstarrungspunkt.

Auch ist die anfänglich besonders tiefe Temperatur, wie sie bei Eisbeuteln unvermeidlich ist, in vielen Fällen schädlich, weil diese tiefe Temperatur zu einem momentanen Kälteschock führt, wodurch der Organismus an der gekühlten Stelle zur Bereitstellung größerer Wärmemengen angeregt wird.

0039443

- 3 -

Einrichtungen zum Temperieren von Körperstellen sind seit langem für therapeutische Zwecke in vielfältiger Art bekannt. Hierzu gehören elektrisch beheizte Decken oder Bandagen mit Flüssigkeitskreislauf als auch mit Flüssigkeiten oder Pasten gefüllte Wärme- oder Kühlbeutel. Letztere sind nicht für kontinuierlichen Langzeitbetrieb geeignet, da sie nach dem Energieaustausch regeneriert werden müssen.

Für eine dauerhafte Kühlung über mehrere Stunden sind derzeit nur Bandagen oder Decken mit Flüssigkeitskreislauf bekannt. Die Kühlung der durch die Bandage strömenden Flüssigkeit (Wasser-Glykolmischung) wird durch thermodynamisch arbeitende Kühlaggregate (Kühlschrank-Prinzip) erreicht (z.B. US-Patentschriften 3 112 792 und 3 211 216).

Alle bekannten Einrichtungen zum Kühlen von Körperstellen sind darauf ausgerichtet, eine bestimmte Temperatur der Bandage einzustellen und diesen Wert konstant zu halten. Weitere Funktionen werden nicht realisiert.

Der vorliegenden Erfindung liegt nun die Aufgabe zugrunde, die geschilderten Nachteile zu überwinden und eine Vorrichtung zu schaffen, mit der gezielt bestimmte Körperteile oder Körperstellen wirkungsvoll gekühlt werden, ohne daß hierdurch ein Kälteschock oder eine Unterkühlung bzw. eine zu starke, für

- 4 -

den Heilungsverlauf ungünstige Kühlung eintreten kann. Ferner soll diese Vorrichtung durch Ermittlung des Wärmeabflusses von der zu untersuchenden Körperstelle im Vergleich zu einem Erfahrungswert oder zu einem zweiten Meßwert von einer gesunden Körperstelle auch eine Diagnose ermöglichen.

Es hat sich nun gezeigt, daß sich diese Aufgabe in überraschend einfacher Weise mit einer Vorrichtung der eingangs genannten Art lösen läßt, die mit Einrichtungen zur Messung der Temperatur und/oder der Wärmeableitung von der behandelten Körperstelle versehen ist und bei der die Wärmeableitung in Abhängigkeit von einer Vergleichstemperatur derart steuerbar ist, daß gerade nur die überschüssige Wärmemenge abgeführt wird, wobei die Kühlung selbsttätig ausschaltbar ist, sobald die Wärmeerzeugung an der behandelten Körperstelle bzw. die Wärmeableitung auf einen Vergleichswert oder Sollwert zurückgegangen ist. Steigt die Temperatur an der behandelten Körperstelle nach einer gewissen Zeit wieder an, wird selbsttätig die Kühlung erneut eingeschaltet.

Die weiteren vorteilhaften Ausbildungen der erfindungsgemäßen Vorrichtung sind in den Unteransprüchen 2 bis 13 erläutert.

Die erfindungsgemäße Vorrichtung eignet sich insbesondere zur Behandlung von rheumatisch entzündeten Gelenken. Dadurch wird

**-5-**

die durch die Entzündung hervorgerufene übermäßige Durchblutung und damit verbundene Erwärmung auf das Normalmaß zurückgeführt. Die Vorrichtung ist jedoch nicht nur zum Kühlen bestimmt, sondern kann darüberhinaus auch die abgeführte Wärmemenge registrieren und aus dem Verlauf der Wärmeaufnahme die Kühlleistung und die Kühldauer bestimmen. Die erfindungsgemäße Vorrichtung kann daher auch für diagnostische Zwecke verwendet werden, wobei aus dem Verlauf der abgeführten Wärmemenge Rückschlüsse auf den Erkrankungszustand und auf den Heilungsprozeß möglich sind.

Die Erfindung wird anhand beiliegender Zeichnungen näher erläutert. Es zeigen in schematischer Vereinfachung

Figur 1 in Aufsicht eine erfindungsgemäße Kühlbandage;
Figur 2 a) und b) in Seitenansicht die Kühlbandage der erfindungsgemäßen Art;
Figur 3 in Aufsicht eine Bandage für die Messung der Vergleichstemperatur;
Figur 4 in Seitenansicht die in Figur 3 gezeigte Bandage;
Figur 5 im Blockschaltbild das Versorgungsgerät und
Figur 6 im Blockschaltbild die elektronische Überwachungseinrichtung.

Die erfindungsgemäße Vorrichtung besteht in der hier beschrie-

-6-

benen Ausführungsart im wesentlichen aus einer Bandage für kranke Körperteile, einer weiteren Bandage zur Messung der Temperatur gesunder Körperteile, einem Versorgungsgerät und einer elektronischen Überwachungseinrichtung.

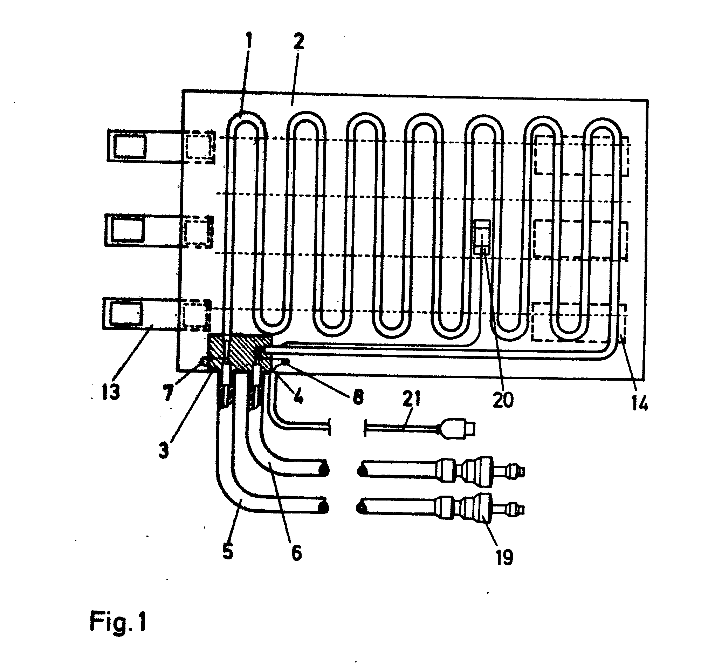

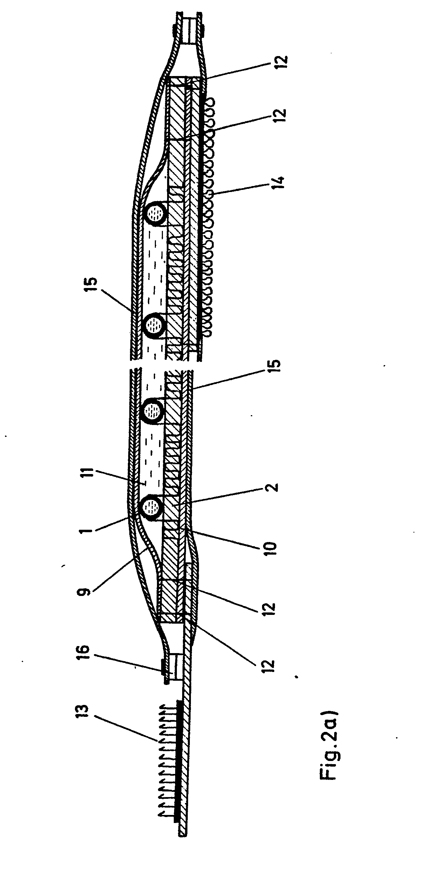

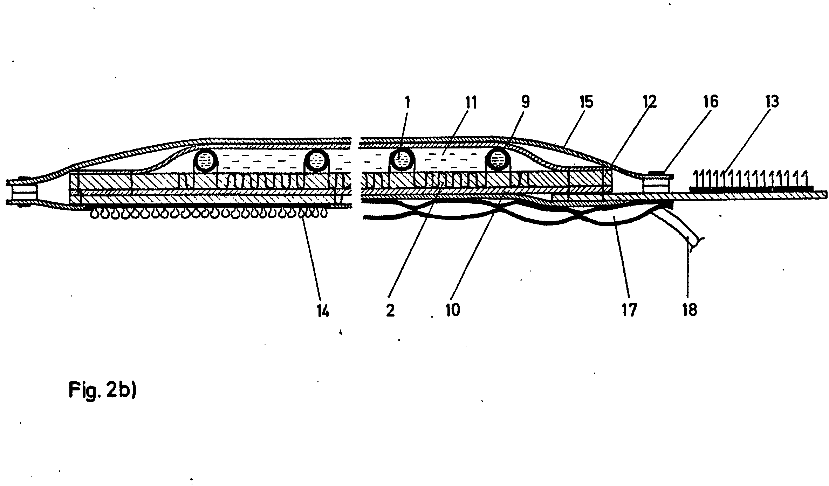

In den Figuren 1, 2 a) und b) wird eine mit einem elastischen, von dem Kühlmittel durchflossenen Kunststoffschlauch 1 versehene Bandage dargestellt. Der Schlauch 1, der z.B. aus Vinylpolymeren besteht, ist quer zur Längsrichtung in einem Abstand vom 2- bis 3-fachen des Schlauchdurchmessers verlegt und auf einem elastischen Gewebe 2 festgenäht. Die beiden Enden des Schlauchs 1 sind in ein Übergangsstück 3 und 4 aus Kunststoff eingeklebt. Auf der anderen Seite der Übergangsstücke 3 und 4 sind flexible Zuleitungsschläuche 5 und 6 zum Versorgungsgerät befestigt. Sie sind gut wärmeisoliert. Dazwischen ragen zwei Thermoelemente 7 und 8 in den Flüssigkeitsstrom, um die Temperatur der Kühlflüssigkeit am Ein- und Auslauf der Bandage messen zu können. Der gesamte Aufbau mit Ausnahme der Zuleitungen ist in ein Kissen 9 bzw. 10 aus elastischen Kunststoffgeweben eingenäht. Auf der als Auflage auf die Haut vorgesehenen Seite des Kühlmittelschlauchs befindet sich das dünne, relativ gut wärmeleitende Gewebe 9 und auf der Rückseite das dickere, wärmeisolierende Gewebe 10. Die Zwischenräume 11 innerhalb der Kühlmittelschlauchanordnung sind vorzugsweise mit einer Wärmeleitpaste ausgefüllt,

- ⅄-

um einen möglichst niedrigen Wärmewiderstand zwischen Kühlmittelschlauch 1 und der Haut zu erreichen. Die Befestigung 12 des Trägergewebes an dem Kissen geschieht an den Seiten, die senkrecht zur beabsichtigten Ausdehnungsrichtung der Bandage liegen. An diesen Seiten sind z.B. drei Klettenverschlüsse 13 und 14 zur Befestigung und Anpassung an das zu behandelnde Körperteil angebracht. Ein Kissenüberzug 15 aus dünnem elastischem Stoff mit Klettenverschluß 16 ist zum Waschen leicht abnehmbar und ermöglicht somit eine hygienische Benutzung auf lange Zeit und gewährleistet angenehme Trageeigenschaften. Auf der Außenseite der Bandage kann ein ausdehnbares Kammersystem 17 vorgesehen werden, das nach dem Anlegen und Verschließen der Bandage, z.B. durch Aufblasen bei 18, an die zu kühlende Körperstelle anpreßbar ist. Die Verbindung der Bandage über Schläuche 5 und 6 mit dem Versorgungsgerät erfolgt mit beidseitig verschließbaren Flüssigkeitskupplungen 19. Um die Hauttemperatur des entzündeten Gelenks zu messen, ist in die Bandage ein Thermoelement 20, wie bei der Vergleichstemperaturmeßstelle eingebaut. Die Meßwerte der drei Thermoelemente 7,8 und 20 werden über ein abgeschirmtes Kabel 21 zum Versorgungsgerät geführt.

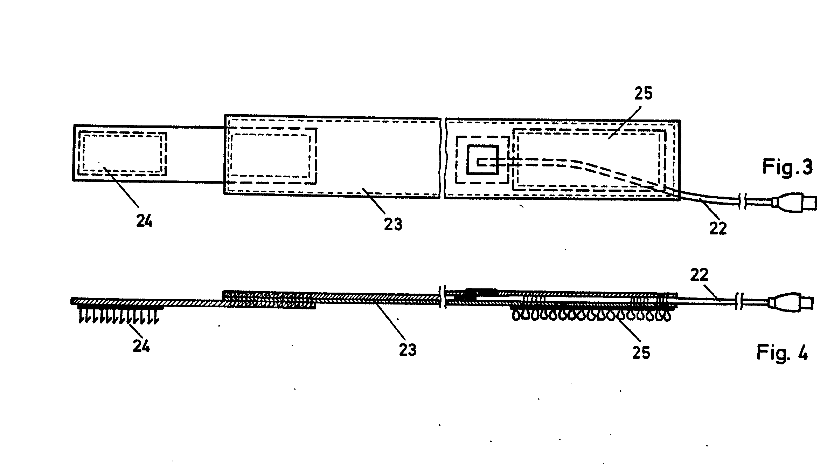

Die Erfassung der Körpertemperatur des gesunden Gelenks geschieht mit der Absicht, die Kühlmitteltemperatur in der Bandage nur geringfügig unter diese Referenztemperatur einzustellen, um eine Gegenreaktion des Organismus bei zu starker Abkühlung zu verhindern. Eine solche Bandage zur Messung der Vergleichstemperatur ist in Figur 3 und 4 dargestellt. Ein Thermoelement 22 ist in eine schmale Bandage, die aus zwei elastischen übereinander angeordneten Geweben 23 besteht, eingenäht. Das Ende des Thermoelements 22 ragt geringfügig aus der unteren Gewebeseite heraus und liegt somit beim Tragen der Bandage auf der Haut. Zur Befestigung der Bandage am Gelenk dient ebenfalls ein Klettenverschluß 24 und 25.

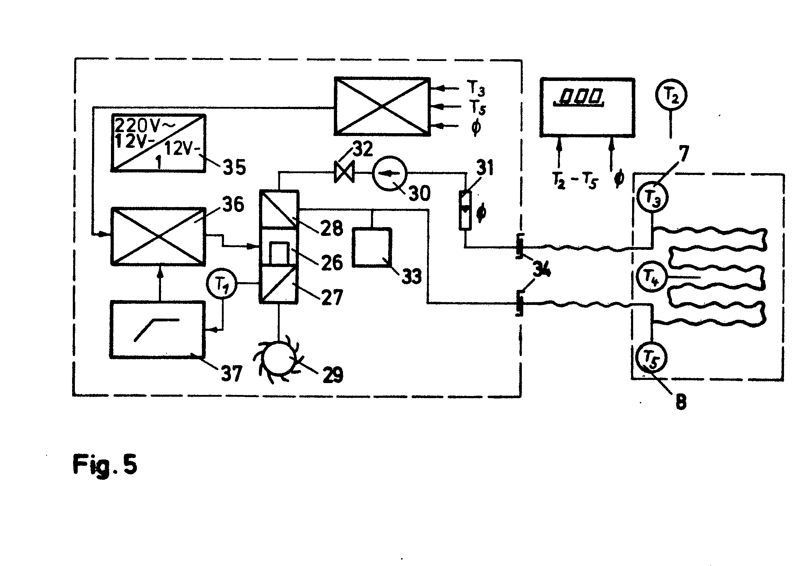

In Figur 5 wird der Aufbau des Versorgungsgerätes gezeigt. In diesem Versorgungsgerät enthalten ist eine Einrichtung zur Erzeugung der Kühlleistung und des Transports des Kühlmittels sowie die Elektronik zur Überwachung und Steuerung der Behandlung. Die Kühlleistung wird mit einem Peltier-Element 26 mit einer elektrischen Leistung von etwa 20 W erzeugt. Das Element 26 ist auf der Warmseite zur Abfuhr der Verlustwärme zwischen einen Luftwärmetauscher 27 und auf der Kaltseite zum Kühlen des Betriebsmediums einen Flüssigkeitswärmetauscher 28 eingebaut. Um den Wärmewiderstand des Luftwärmetauschers 27 zu verringern, ist ein Ventilator 29 vorgesehen. Der Flüssigkeitskreislauf im Versorgungsgerät enthält ferner eine Kreiselpumpe 30 mit einer Leistung von 3 l/min., für den Kühlmitteltransport einen optoelektronischen Durchflußmesser 31 zur Errechnung der abgeführten Wärmemenge, ein elektrisch betriebenes Ventil 32 zum Einstellen der Durchflußrate und einen Vorratsbehälter 33. Die Verbindung zu den Schläuchen der Bandage erfolgt durch selbstverriegelnde Flüssigkeitskupplungen 34. Betrieben wird das Peltier-Element 26 mit einer Gleichspannung von 12 V aus einem geregelten Netzteil 35 und 36. Zum Schutz vor Überhitzung der Warmseite des Peltier-Elements 26 wird die Temperatur an dieser Stelle mit einem Thermoelement T1 gemessen. Der Komparator 37 vergleicht diesen Wert mit einem vorgegebenen Maximalwert und schaltet das Netzteil 35 und 36 beim Überschreiten dieses Wertes ab.

Die erfindungsgemäße Vorrichtung ermöglicht auch die Festlegung einer Solltemperatur, indem die Temperatur der Bandage für kranke Körperteile kontinuierlich erniedrigt wird und durch Messung der Temperatur an einem vergleichbaren gesunden Körperteil die Reaktion des Organismus auf die Kühlbehandlung registriert wird. Wird diese Reizschwelle erkannt, kann die Temperatur der Kühlbandage entsprechend erhöht werden, um eine Gegenreaktion des Organismus gegen die Kühlbehandlung zu vermeiden. Hierfür muß jedoch im Versorgungsgerägt ein Sägezahngenerator vorgesehen sein, der die Temperatur der Kühlbandage kontinuierlich erniedrigt. Ferner wird eine Elektronik benötigt, mit der die Temperatursignale aus der Vergleichstemperaturmeßstelle verarbeitet werden und die in Abhängigkeit von diesen Meßwerten den Sägezahngenerator steuert.

- 10 -

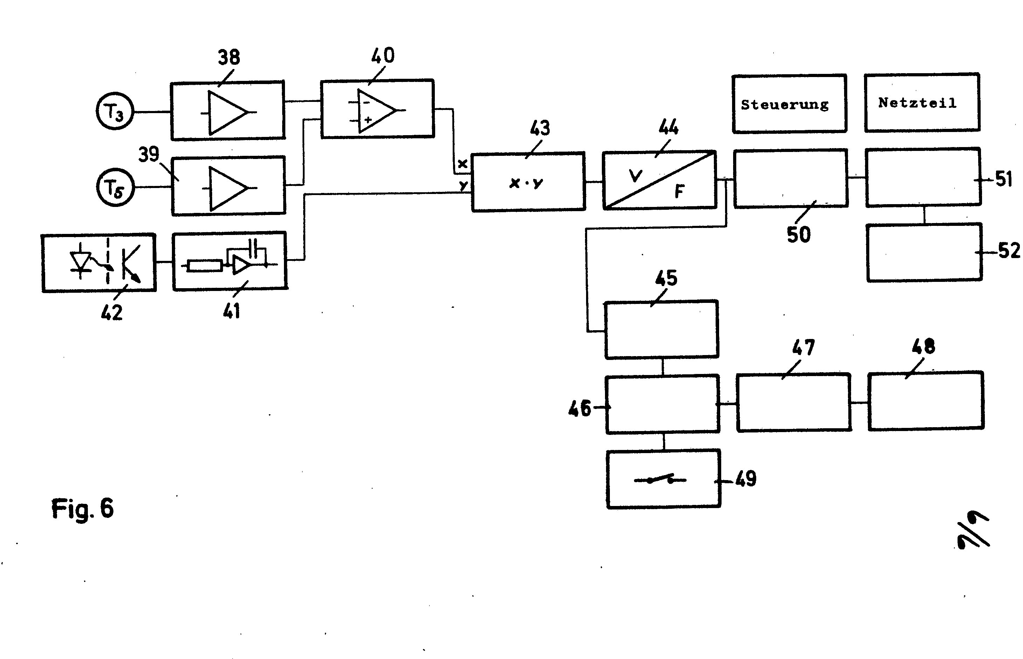

Die Elektronik zur Überwachung der Behandlung ist als Blockschaltbild in Figur 6 dargestellt. Zur Bestimmung der von der Bandage abgeführten Wärmemenge werden die Temperaturen $T_3$ und $T_4$ mit Thermoelementen 7 und 8 gemessen und die Signale bei 38 und 39 vorverstärkt. Anschließend wird die Temperaturdifferenz bei 40 gebildet. Dieser Wert wird mit dem über eine definierte Zeit aufintegrierten Ausgangssignal 41 des optoelektronischen Durchflußmessers 42 bei 43 multipliziert. Das Ergebnis ist die pro Zeit abgeführte Wärmemenge. Um zu entscheiden, wann die Behandlung beendet werden kann, wird die Differenz zwischen zwei im Abstand von mehreren Minuten aufeinanderfolgenden Messungen gebildet. Dies geschieht digital, indem die Ausgangsspannung des Multiplizierers 43 und 44 in eine Frequenz umgewandelt und in einem Vor-Rückwärtszähler 45 die Differenz gebildet wird. Das Ergebnis wird anschließend mit einem vorgegebenen Grenzwert verglichen, und zwar durch den Komparator 46, den Speicher 47 und den Codierschalter 48. Sobald der Grenzwert unterschritten wird, schaltet der Regler 49 das Netzgerät zur Versorgung des Peltier-Elements 26 ab. Zur graphischen Darstellung des Verlaufs der Kühlbehandlung werden die gemessenen Werte der pro Zeit abgeführten Wärmemenge digital bei 50 und 51 gespeichert und mit einer Leuchtdiodenmatrix 52 angezeigt.

0039443

- 1 -

V-64.053-01/57/79

CASCH/KRI                            18. April 1980

PETER STEINGRAF, BAD HOMBURG

Patentansprüche

1. Vorrichtung zur Kühlung von Körperstellen oder Körperteilen im Rahmen therapeutischer oder diagnostischer Maßnahmen, bestehend im wesentlichen aus einer von einem Wärmetransportmedium durchflossenen Auflage oder Bandage, einem Kühlaggregat und aus Meß- und Regelungseinrichtungen zur Steuerung des Wärmetransportes, dadurch gekennzeichnet, daß Einrichtungen (7,8,20) zur Messung der Temperatur und/oder der Wärmeableitung von der behandelten Körperstelle vorgesehen sind und daß die Wärmeableitung in Abhängigkeit von einer Vergleichstemperatur derart steuerbar ist, daß gerade nur die überschüssige Wärmemenge abgeführt wird, wobei die Kühlung selbsttätig ausschaltbar ist, sobald die Wärmeerzeugung an der behandelten Körperstelle bzw. die Wärmeableitung auf einen Vergleichswert oder Sollwert zurückgegangen ist.

2. Vorrichtung nach Anspruch 1, dadurch gekennzeichnet, daß der Vergleichswert oder Sollwert mit Hilfe einer zusätzlichen, an einem äquivalenten, jedoch gesunden Körperteil anlegbaren Temperaturmeßelektrode (22) ermittelbar ist.

3. Vorrichtung nach Anspruch 1, dadurch gekennzeichnet, daß als Vergleichswert oder Sollwert ein Erfahrungswert vorgebbar ist.

4. Vorrichtung nach Anspruch 1 oder 2, dadurch gekennzeichnet, daß die Differenz zwischen der Temperatur des Wärmetransportmediums am Einlaß in die Bandage bzw. in die Auflage und der Normaltemperatur des äquivalenten Körperteils auf einen Maximalwert von 1 bis 10 $^{\circ}$C, vorzugsweise 1 bis 5 $^{\circ}$C, begrenzt ist.

5. Vorrichtung nach einem der Ansprüche 1 bis 4, dadurch gekennzeichnet, daß zur Ermittlung der Wärmeableitung die Einlauf- und Auslauftemperatur des Wärmetransportmediums an der Auflage bzw. Bandage und die Durchflußrate ermittelbar sind.

6. Vorrichtung nach einem der Ansprüche 1 bis 5, dadurch gekennzeichnet, daß zur Feststellung und Überwachung des Erkrankungszustandes sowie des Heilungsprozesses die Wärmeableitung selbsttätig in regelmäßigen Zeitabständen unterbrochen und der Temperaturanstieg an der behandelten Körperstelle ermittelt wird.

7. Vorrichtung nach einem der Ansprüche 1 bis 5, dadurch gekennzeichnet, daß die abgeführte Wärmemenge kontinuierlich
meßbar ist und daß bei Verringerung der Wärmeableitung pro
Zeiteinheit auf einen Vergleichs- oder Sollwert die Kühlung
abschaltbar ist.

8. Vorrichtung nach einem der Ansprüche 1 bis 7, dadurch gekennzeichnet, daß das Versorgungsgerät thermoelektrisch arbeitende Peltier-Elemente (26) enthält.

9. Vorrichtung nach einem der Ansprüche 1 bis 8, dadurch gekennzeichnet, daß die Auflage oder Bandage mehrere Schichten
aufweist, nämlich eine dünne Lage eines Gewebes oder einer
Folie (9,15), welche auf der Körperstelle aufliegt, ferner
eine von dem Wärmetransportmedium durchflossene, ein oder
mehrere Leitungs- oder Kammersysteme enthaltende Schicht
(2,11) und eine außen aufgebrachte flexible Wärmeisolationsschicht (10) sowie gegebenenfalls eine zwischen die Schichten eingefügte, mechanisch stabile, flexible Trägerschicht.

10. Vorrichtung nach Anspruch 9, dadurch gekennzeichnet, daß in
die von dem Wärmetransportmedium durchflossene Schicht (11) zwischen den einzelnen Leitungen (1) oder Kammern eine wärmeleitfähige Paste eingefügt ist.

11. Vorrichtung nach einem der Ansprüche 1 bis 10, dadurch gekennzeichnet, daß der Auflage oder Bandage zusätzlich gasförmige oder dampfförmige Medien zuführbar sind, die auf der dem Körper zugewandten Seite durch Düsen oder Poren hindurch zu der Auflagefläche hinleitbar sind.

12. Vorrichtung nach einem der Ansprüche 1 bis 11, dadurch gekennzeichnet, daß in die Auflage oder Bandage zusätzliche Elektroden eingearbeitet sind, mit denen für therapeutische Maßnahmen im Bereich der temperierten Körperstelle elektrostatische Gleich- oder Wechselfelder erzeugbar sind.

13. Vorrichtung nach einem der Ansprüche 1 bis 12, dadurch gekennzeichnet, daß diese auf der Außenseite, d.h. auf der der Auflagefläche gegenüberliegenden Seite der Bandage, mit einem ausdehnbaren Kammersystem (17) versehen ist, das derart ausgebildet und angeordnet ist, daß nach dem Anlegen und Verschließen der Bandage die Auflagefläche durch Aufblasen des Kammersystems an die zu kühlende Körperstelle anpreßbar ist.

Fig. 1

Fig.2a)

Fig. 2b)

1  11  9  15  12  16  13

14  2  10  17  18

3/6

0039443

25

24

23

22

**Fig.3**

22

24

23

25

**Fig. 4**

2/4

0039443

Fig. 5

0039443

Fig. 6

0039443

| | Europäisches Patentamt | **EUROPÄISCHER RECHERCHENBERICHT** | Nummer der Anmeldung<br>EP 81 10 3029 |

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe, soweit erforderlich. der maßgeblichen Teile | betrifft Anspruch | A 61 F 7/00<br>A 61 B 5/00 |
| | <u>US - A - 3 007 473</u> (JACKSON, SHULL) | 1,3 | |
| | * Figuren 1,5; Spalte 2, Zeile 66 bis Spalte 7, Zeile 73 * | | |
| | <u>FR - A - 2 366 008</u> (CHATTANOOGA PHARMACAL Co.) | 1,3,9 | |
| | * Figuren 1,2,6; Seite 3, Zeile 33 bis Seite 5, Zeile 2; Seite 5, Zeile 22 bis Seite 6, Zeile 20; Seite 12, Zeile 2 bis Seite 13, Zeile 10 * | | |
| | & DE - A - 2 743 919 | | RECHERCHIERTE SACHGEBIETE (Int Cl ) |
| | <u>DE - B - 1 053 537</u> (SIEMENS-REINIGER-WERKE) | 1,8 | A 61 F<br>A 61 M<br>A 61 B |
| | * Figur 1; Spalte 1, Zeilen 1-22 * | | |
| | <u>US - A - 3 867 939</u> (MOORE et al.) | 9 | |
| | * Figuren 1,2; Spalte 3, Zeile 53 bis Spalte 6, Zeile 65 * | | |
| | <u>US - A - 4 118 946</u> (TUBIN) | 10 | |
| | * Figuren 1-3; Spalte 2, Zeilen 19-30 * | | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 03.08.1981 | HERBELER |

EPA form 1503.1 06.78